# EUROPEAN PATENT APPLICATION

(11) **EP 0 976 750 A1**
(43) Date of publication of application: **02.02.2000**
(21) Application number: 98900380.1
(22) Date of filing: 16.01.1998
(51) Int. Cl.: C07D 473/18

(54) **NOVEL Z-VALACYCLOVIR CRYSTALS**

(30) Priority: 17.01.1997 JP 678597
(71) Applicant: Ajinomoto Co., Inc., Tokyo 104 (JP)
(72) Inventor: TAKEDA, Hideo, Ajinomoto Co., Inc., Kawasaki-shi, Kanagawa-ken 210 (JP); KATAYAMA, Satoshi, Ajinomoto Co., Inc., Kawasaki-shi, Kanagawa-ken 210 (JP); INEYAMA, Takashi, Ajinomoto Co., Inc., Yotsukaichi-shi, Mie-Ken 510 (JP); KISHISHITA, Akihiro, Ajinomoto Co., Inc., Kawasaki-shi, Kanagawa-ken 210 (JP); NAGASHIMA, Kazutaka, Ajinomoto Co., Inc., Kawasaki-shi, Kanagawa-ken 210 (JP)
(74) Representative: Nicholls, Kathryn Margaret
(86) International application number: JP9800128
(87) International publication number: WO9831683

(57) **Abstract**

Crystals of 2-[(2-amino-1,6-dihydro-6-oxo-9H-purine-9-yl)methoxy]ethyl N-[(benzyloxy)carbonyl]-L-valinate, having peaks at angles of diffraction of at least 15.3° , 21.3° , 23.8° and 27.2° (2θ, CuKα rays) in the diffraction X-ray in the determination by the powder X-ray diffraction method, are provided. These catalysts are substantially not hygroscopic and have a high purity and a low impurity content and, therefore, useful as intermediates for Valaciclovir used as an antiviral drug.

## Description

### Background of the Invention

The present invention relates to new Z-Valaciclovir crystals, which are a synthetic precursor of Valaciclovir used as a prodrug of acyclovir widely used as an antiviral drug in the world, and approved as a remedy for herpes, shingles and the like at present.

9-[(2-Hydroxyethoxy)methyl]guanine known as acyclovir has a strong antiviral activity particularly against viruses in the herpes group, and now widely used as a remedy for herpes, shingles and the like in the world. However, since acyclovir has only low water solubility and oral absorbency, it has a problem that it must be administered in a large amount in order to keep the antiviral concentration in the blood plasma on an effective level.

After investigations made for the purpose of improving the properties of this compound, it was found that amino acid esters, particularly L-valine ester, are the best in regard to the oral absorbency [Japanese Patent Unexamined Published Application (hereinafter referred to as "J. P. KOKAI") No. Sho 64-68373 (European Patent Publication No. 596542 A), and Antiviral Chemistry & Chemotherapy (1992, 3 (3), pages 157-164]. L-Valine ester of acyclovir (Valaciclovir) was already approved as a prodrug of acyclovir in the U.S.A and the United Kingdom, and is expected to be used as a remedy for herpes in place of acyclovir.

For the production of Valaciclovir, a process wherein the protective group of 2-[(2-amino-1,6-dihydro-6-oxo-9H-purine-9-yl)methoxy]ethyl N-[(benzyloxy)carbonyl]-L-valinate (Z-Valaciclovir), obtained by reacting acyclovir with Z-L-valine, is removed by a reduction is known (J. P. KOKAI No. Sho 64-68373). Incidentally, Valaciclovir used as a medicine is requested to have as low as possible impurity content, and Z-Valaciclovir having higher purity and quality has been demanded to be used as the precursor.

Z-Valaciclovir used hitherto was only in the form of a white solid (J. P. KOKAI No. Sho 64-68373), which has problems in the quality and handling. After investigations, the inventors have found that dry crystals, obtained by condensing Z-L-valine with acyclovir as the starting materials, then crystallizing the product in an ordinary manner, and filtering and drying the product, have a low purity and they are hygroscopic and have serious problems in the quality.

### Disclosure of the Invention

An object of the present invention is to provide new crystals of Z-Valaciclovir having a high purity and a low hygroscopicity, to be used as a precursor for synthesizing Valaciclovir useful as a remedy for herpes.

Another object of the present invention is to provide a process for producing a new crystalline form of Z-Valaciclovir.

Still other objects of the invention will be apparent from the description and Examples given in this specification.

After intensive investigations made for the purpose of attaining the above-described objects, the inventors have found that new Z-Valaciclovir crystals having a high purity and a low hygroscopicity can be obtained by a process which comprises adding a poor solvent to the reaction solution obtained by the condensation, to obtain a suspension, heating the suspension to obtain a solution again, cooling the solution to form crystals and controlling the conditions for the crystallization, drying and the like when separating the solid from the liquid and drying the solid are further conducted. The present invention has been completed on the basis of this finding.

Namely, the present invention provides crystals of 2-[(2-amino-1,6-dihydro-6-oxo-9H-purine-9-yl)methoxy]ethyl N-[benzyloxy)carbonyl)-L-valinate characterized by having peaks at angles of diffraction of at least 15.3° , 21.3° , 23.8° and 27.2° in the diffraction X-ray in the determination by the powder X-ray diffraction method with CuKα rays.

The present invention also provides a process for producing the above-described crystals, which comprises esterifying N-[(benzyloxy)carbonyl]-L-valine with acydovir in the presence of a coupling agent and a basic catalyst in a good solvent, removing by-products and unreacted compounds to obtain a solution dissolving Z-Valaciclovir, then adding 50 to 4,000 parts by weight of a poor solvent to 100 parts by weight of the solution to form and thereby to suspend Z-Valaciclovir crystals, heating the obtained suspension to completely redissolve the formed crystals, cooling the obtained solution to form crystals of Z-Valaciclovir, washing the crystals with the above-described poor solvent and drying the crystals at 55 to 80°C.

### Brief Description of the Drawings

Fig. 1 shows the powder X-ray diffraction of Z-Valaciclovir crystals (A-type crystals) of the present invention obtained in Example 1.
Fig. 2 shows the powder X-ray diffraction of Z-Valaciclovir crystals (B-type crystals) obtained in Comparative Example 1.
Fig. 3 shows the changes in water content with time observed by keeping Z-Valaciclovir crystals (A-type crystals) of the present invention and Z-Valaciclovir crystals (B-type crystals) obtained in the Comparative Example in an air-conditioned room.

### Best Mode for Carrying Out the Invention

The crystals of the present invention can be obtained by the following procedure: Dicyclohexylurea in a reaction solution obtained by a process described in J. P. KOKAI No. Sho 64-68373 (European Patent Publication No. 596542 A) is removed by the filtration. Then a suitable poor solvent is added to the filtrate. The resultant suspension is once heated to obtain a solution, which is cooled to form crystals. The crystals are separated and thereby isolated, and then dried to obtain the intended crystals. The contents of the specification of J. P. KOKAI No. Sho 64-68373 (European Patent Publication No. 596542 A) are to be included in the specification of the present invention.

When the amount of the poor solvent added in this step is insufficient or the drying temperature is low, crystals which are industrially useless are obtained instead of the intended crystals. Therefore, the amount of the poor solvent is preferably 50 to 4,000 parts by weight for 100 parts by weight of the Z-Valaciclovir solution. The drying temperature is preferably 55 to 80°C, more preferably about 58 to 65°C and most preferably about 60°C.

If necessary, unreacted dicyclohexylcarbodiimide may be decomposed by adding acetic acid to the reaction system prior to the filtration of dicyclohexylurea, and then the crystals are obtained by the filtration, crystallization and isolation.

The poor solvents usable herein include water, alcohols (such as methanol, ethanol, denatured ethanol, and isopropyl alcohol) and a mixture of water and a lower alcohol in any proportion. Among them, methanol, ethanol, isopropyl alcohol and mixtures of one of them with water are preferred. A mixture of water and isopropyl alcohol is more preferred. A mixture of water and isopropyl alcohol in a volume ratio of about 1:1 is the most preferred.

The drying method is not particularly limited, and an air dryer, fluid dryer, vacuum dryer, spray dryer, micron dryer or the like is usable.

Concretely, the process of the present invention for producing the crystals can be conducted in, for example, the following manner:

N-[(benzyloxy)carbonyl]-L-valine is esterified with acyclovir of the following chemical formula (I) (wherein hydroxyl group and amino group may be protected with suitable protecting groups) with a coupling agent such as N,N'-dicyclohexylcarbodiimide in the presence of a basic catalyst such as dimethylaminopyridine in a good solvent such as pyridine or dimethylformamide (dimethylformamide is preferred):

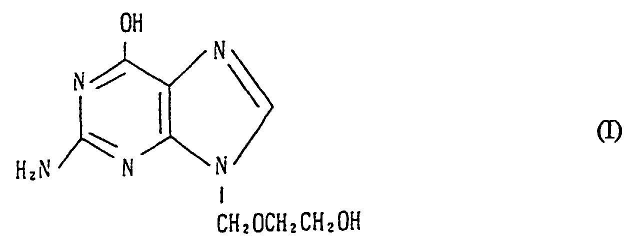

More concretely, N-[(benzyloxy)carbonyl]-L-valine and a basic catalyst such as dimethylaminopyridine are dissolved in a solvent such as pyridine or dimethylformamide, then acyclovir of chemical formula (I) is added to the resultant solution to obtain a suspension, and a solution of a coupling agent such as N,N'-dicyclohexylcarbodiimide in the same solvent as that used above is dropped into the suspension to conduct the esterification reaction. The reaction temperature is preferably 0 to 30°C.

Then dicyclohexylurea is removed from the reaction solution by the filtration to obtain a solution of Z-Valaciclovir. It is preferred that unreacted dicyclohexylcarbodiimide is decomposed by addinng acetic acid to the reaction system prior to the removal of dicyclohexylurea by the filtration.

In the present invention, 50 to 4,000 parts by weight of the poor solvent is added to 100 parts by weight of the solution of Z-Valaciclovir obtained by the above-described process to form and thereby to suspend Z-Valaciclovir crystals, and then the obtained suspension is heated (to about 65°C) to completely redissolve the formed crystals. Then the solution is preferably gradually cooled from 65°C to 60°C for the duration of 20 to 60 minutes (preferably about 30 minutes), and then from 60°C to 53°C for the duration of 40 to 120 minutes (preferably about 60 minutes) to form Z-Valaciclovir crystals. The product is aged at 53°C for 30 to 120 minutes (preferably about 60 minutes) and then cooled to room temperature or below, the crystals are taken by the filtration and washed with the same poor solvent as that used before to obtain wet crystals, which are dried at 55 to 80°C to obtain the crystals of the present invention.

The following Referential Example and Examples will further illustrate the present invention:

### Referential Example: Preparation of solution of 2-[(2-amino-1,6-dihydro-6-oxo-9H-purine-9-yl)methoxy]ethyl N-[(benzyloxy)carbonyl]-L-valinate (Z-Valaciclovir) in DMF before the crystallization:

N-[(Benzyloxy)carbonyl]-L-valine (Z-L-valine; 41.1 kg 163.6 mol) and dimethylaminopyridine (DMAP; 1.67 kg, 13.6 mol) were dissolved in dimethylformamide (DMF) cooled to 5°C. Acyclovir (corresponding to 2/3 hydrate; 32.34 kg, 136.3 mol) was suspended in the obtained solution. A 60 % solution (4.7 kg, 13.6 mol) of dicyclohexylcarbodiimide (DCC) in DMF was added to the obtained suspension. The resultant mixture was stirred for about 30 hours while 60 % solution (70.3 kg, 204.5 mol) of DCC in DMF was slowly dropped thereinto for a period of about 30 hours. After the completion of the dropping, the reaction solution was analyzed by the high-performance liquid chromatography to find that the amount of starting acyclovir still remaining therein had been already reduced to about 3 %. After stirring at 5°C for 10 hours for completing the reaction, the reaction solution was analyzed by the high-performance liquid chromatography to find that the reaction had been completed almost quantitatively. Acetic acid (4.1 kg, 68.2 mol) was added to the reaction solution to decompose unreacted DCC. White solids in the reaction solution were filtered out, and the filter cake was washed with DMF (12 L) to obtain about 200 L of 24 wt. % solution of the intended product in DMF.

### Example 1 Preparation of dry crystals A:

Dicyclohexylurea was filtered out of the reaction solution obtained in the Referential Example. 351 ml of a mixed solution of isopropyl alcohol and water (mixing volume ratio: 1:1) was added to 50 g of the 24 wt. % solution of Z-Valaciclovir to obtain a suspension (Z-Valaciclovir concentration: 3.3 wt. %), which was heated to 65°C to completely dissolve the slurry. The obtained solution was cooled from 65°C to 60°C for the duration of 30 minutes and then from 60°C to 53°C for the duration of one hour. In this step, white crystals began to be formed at 53°C. After aging at that temperature for one hour, the reaction mixture was cooled to 36°C for the duration of 15 hours and then to 10°C. The crystals were taken by the filtration and then washed with 55 ml of a mixed solution of isopropyl alcohol and water (mixing volume ratio: 1:1) to obtain the wet crystals, which were dried at 60°C in vacuum to obtain dry crystals A.

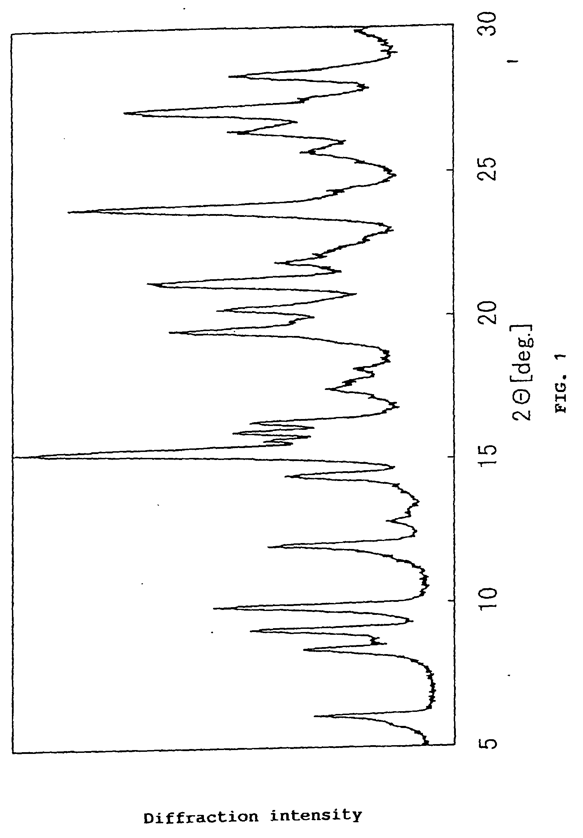

Dry crystals A obtained as described above were tested by the powder X-ray diffraction method with CuKα rays. Fig 1 shows the results of the powder X-ray diffraction thus obtained.

It is apparent from Fig. 1 that dry crystals A had characteristic diffraction peaks at 15.3° , 27.2° , 23.8° and 21.3° .

These crystals will be referred to as "A type crystals" hereinafter.

### Comparative Example 1 Preparation of dry crystals B:

Dicyclohexylurea was removed by the filtration from the reaction solution prepared in the Referential Example 1. 351 ml of a mixed solution of isopropyl alcohol and water (mixing volume ratio: 1:1) was added to 50 g of the solution containing 24 wt % of Z-Valaciclovir to obtain a suspension (Z-Valacidovir concentration: 3.3 wt. %). The suspension was heated to 65°C to completely dissolve the slurry which was then gradually cooled from 65°C to 60°C for the duration of 30 minutes and then from 60°C to 53°C for the duration of one hour. In this step, white crystals began to be formed at 53°C. After aging at that temperature for one hour, the reaction mixture was cooled to 36°C for the duration of 15 hours and then to 10°C. The crystals were taken by the filtration and then washed with 55 ml of a mixed solution of isopropyl alcohol and water (mixing volume ratio: 1:1) to obtain the wet crystals, which were dried at 50°C in vacuum to obtain dry crystals B.

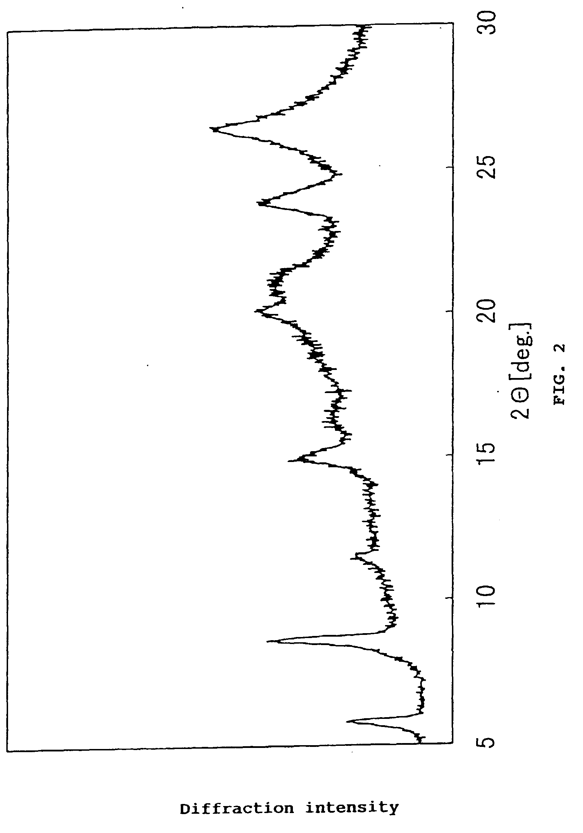

Dry crystals B obtained as described above were tested by the powder X-ray diffraction method with CuKα rays. Fig. 2 shows the results of the powder X-ray diffraction thus obtained.

It is apparent from Fig. 2 that dry crystals B do not have diffraction peaks at angles of diffraction of any of 15.3° , 27.2° and 21.3° which crystals A have. Thus, dry crystals B can be dearly differentiated from crystals A. These crystals will be referred to as "B-type crystals" hereinafter.

### Comparative Example 2 Preparation of dry crystals C:

Dicyclohexylurea was removed by the filtration from the reaction solution prepared in the Referential Example. 255 ml of a mixed solution of isopropyl alcohol and water (mixing volume ratio: 1:1) was added to 104 g of the solution containing 24 wt % of Z-Valaciclovir to obtain a suspension (Z-Valaciclovir concentration: 7.5 wt. %). The suspension was heated to 65°C to completely dissolve the slurry, which was then gradually cooled from 65°C to 60°C for the duration of 13 minutes. After aging at that temperature for one hour, the reaction mixture was gradually cooled to 10°C for the duration of 10 hours. White crystals began to be formed at 59.6°C. The crystals were taken by the filtration and then washed with 112 ml of a mixed solution of isopropyl alcohol and water (mixing volume ratio: 1:1) to obtain the wet crystals, which were dried at 50°C in vacuum to obtain dry crystals C.

Dry crystals C obtained as described above were analyzed by the powder X-ray diffraction method to obtain the same pattern as that of B-type crystals.

### Comparative Example 3 Preparation of dry crystals D:

Dicyclohexylurea was removed by the filtration from the reaction solution prepared in the Referential Example. 255 ml of a mixed solution of isopropyl alcohol and water (mixing volume ratio: 1:1) was added to 104 g of the solution containing 24 wt % of Z-Valaciclovir to obtain a suspension (Z-Valaciclovir concentration: 7.5 wt. %). The suspension was heated to 65°C to completely dissolve the slurry, which was then gradually cooled from 65°C to 60°C for the duration of 13 minutes. After aging at that temperature for one hour, the reaction mixture was gradually cooled to 10°C for the duration of 10 hours. White crystals began to be formed at 59.6°C. The crystals were taken by the filtration and then washed with 112 ml of a mixed solution of isopropyl alcohol and water (mixing volume ratio: 1:1) to obtain the wet crystals, which were dried at 60°C in vacuum to obtain dry crystals D.

Dry crystals D obtained as described above were analyzed by the powder X-ray diffraction method to obtain the same pattern as that of B-type crystals.

### Example 2 Determination of properties of dry crystals:

The hygroscopicity, purity and impurity content of the obtained dry crystals (A-type crystals and B-type crystals) were determined by the following methods:

### (1) Hygroscopicity:

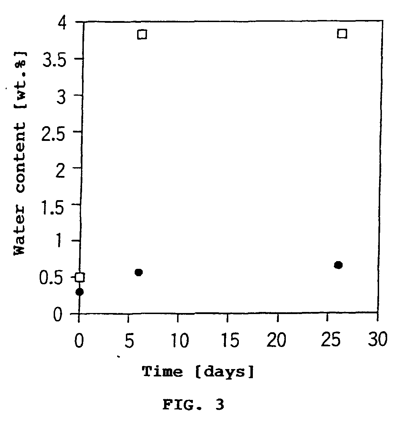

1 g of each of A-type crystals and B-type crystals was placed in a glass Petri dish and kept in an air-conditioned room kept at a temperature of 25°C and at a relative humidity of 35 % to determine the change in water content with time. The results are shown in Fig. 3. In Fig. 3, "●" represents the results obtained when A-type crystals were used at the start of the determination and "□" represents the results obtained when B-type crystals were used. It is apparent from Fig. 3 that the water content of A-type crystals were unchanged for a long time, while B-type crystals absorbed water and were unstable.

After 26 days in the hygroscopicity test, the X-ray diffraction of the crystals was determined by the powder method to find no change in the type of the crystals..

### (2) Purity of crystals (Z-Valaciclovir content)

The purity of the crystals was determined by the high-performance liquid chromatography to find that the purity of A-type crystals was 99.1 wt. % and that of B-type crystals was 94.9 wt. %.

### (3) Impurity content:

According to the high-performance liquid chromatographic analysis, the amount of N,N-dimethylformamide which was one of the impurities was 0.05 wt. % in A-type crystals and 0.18 wt. % in B-type crystals.

It is apparent from the recults of the determination of the properties described above that A-type crystals of the present invention were substantially not hygroscopic and had a high purity and only a low impurity content unlike B-type crystals and that A-type crystals are, therefore, useful.

In the production of Z-Valacidovir which is an extremely important intermediate for Valaciclovir, the quality of the product was remarkably improved by the finding of new Z-Valaciclovir crystals.

## Claims

1. Crystals of 2-[(2-amino-1,6-dihydro-6-oxo-9H-purine-9-yl)methoxy]ethyl N-[(benzyloxy)carbonyl]-L-valinate having peaks at angles of diffraction of at least 15.3° , 21.3° , 23.8° and 27.2° (2θ, CuKα rays) in the diffraction X-ray in the determination by the powder X-ray diffraction method.

2. A process for producing the crystals according to claim 1, which comprises esterifying N-[(benzyloxy)carbonyl]-L-valine with acyclovir in the presence of a coupling agent and a basic catalyst in a good solvent, removing by-products and unreacted compounds to obtain a solution dissolving Z-Valaciclovir, then adding 50 to 4,000 parts by weight of a poor solvent to 100 parts by weight of the solution to form and thereby to suspend Z-Valaciclovir crystals, heating the obtained suspension to completely redissolve the formed crystals, cooling the obtained solution to form crystals of Z-Valaciclovir, washing the crystals with the above-described poor solvent and drying the crystals at 55 to 80°C.

3. The process according to claim 2, wherein the coupling agent is N,N'-dicyclohexylcarbodiimide.

4. The process according to claim 2, wherein the poor solvent is selected from the group consisting of water, lower alcohols and mixtures of them.

5. The process according to claim 4, wherein the poor solvent is a mixture of water and isopropyl alcohol.

6. The process according to claim 5, wherein the poor solvent is a mixture of water and isopropyl alcohol in a volume ratio of about 1:1.

7. The process according to claim 2, wherein the suspension is heated to about 65°C to completely redissolve the formed crystals.
